# EUROPÄISCHE PATENTSCHRIFT

(11) **EP 1 061 069 B1**
(45) Veröffentlichungstag und Bekanntmachung des Hinweises auf die Patenterteilung: **04.06.2003**
(21) Anmeldenummer: 00108883.0
(22) Anmeldetag: 26.04.2000
(51) Int. Cl.: C07C 275/00, C07C 231/02, C08G 18/38, C08G 18/80, C09D 175/04

(54) **Urethanisierte Beta-Hydroxyalkylamid-Verbindung, ein Verfahren zu ihrer Herstellung sowie deren Verwendung zur Herstellung von Pulverlacken**
Urethanised beta-hydroxyalkylamide compounds, process for their preparation as well as their use in the preparation of powder coatings
Composés uréthanisés a base de béta-hydroxyalkylamide, procédé pour leur préparation ainsi que leur utilisation pour la préparation de revetements en poudre

(30) Priorität: 04.06.1999 DE 19925543
(43) Veröffentlichungstag der Anmeldung: 20.12.2000
(73) Patentinhaber: Degussa AG, 40474 Düsseldorf (DE)
(72) Erfinder: Wenning, Andreas, Dr., 48301 Nottuln (DE)

(56) Entgegenhaltungen:
- EP-A- 0 322 834
- EP-A- 0 698 629
- EP-A- 0 789 043

## Beschreibung

Die Erfindung betrifft urethanisierte β-Hydroxyalkylamid-Verbindungen, ein Verfahren zu ihrer Herstellung, deren Verwendung zur Herstellung hochreaktiver Pulverlacke sowie die Pulverlacke selbst.

Pulverlacke auf Basis Trigylycidylisocyanurat (TGIC) und säurefunktionellen Polyestern ergeben korrosionbeständige und wetterstabile Pulverbeschichtungen. Die EP 0 536 085 beschreibt jedoch, dass zur Herstellung des TGIC in fester Form teure Verfahren oder ein relativ großer und somit ebenfalls teurer Reinigungsaufwand erforderlich sind. Zudem wird TGIC von der Europäischen Gemeinschaft als Mutagen der Kategorie II (,,sollte als erbgutverändernd angesehen werden") eingestuft und ist seit dem 31. Mai 1998 als giftig zu kennzeichnen.

Toxikologisch unbedenklich und zugleich auch reaktiver sind β-Hydroxyalkylamide als Vernetzer. In den Patentschriften US 4,076,917 und US 4,101,606 werden β-Hydroxyalkylamide mit Polymeren, die mindestens eine carboxyl- oder Anhydridfunktion aufweisen, insbesondere mit Polyacrylaten zu Pulverlacken kombiniert. Die EP 0 322 834 beschreibt wärmehärtende Pulverlacke, die aus Säuregruppen enthaltenden Polyestern und β-Hydroxyalkylamiden zusammengesetzt sind. Diese Beschichtungen mit β-Hydroxyalkylamid-Vernetzer sind hochwetterstabil, sehr flexibel, hart und chemikalienresistent. Für viele Anwendungen, etwa im Bereich der Sanitärindustrie oder bei der Beschichtung von Laborgeräten, reicht die Chemikalienbeständigkeit jedoch nicht aus.

Die Aufgabe der vorliegenden Erfindung war es daher, neue Vernetzer zu finden, die in Kombination mit carboxylgruppenhaltigen Polymeren zu Pulverlacken verarbeitet werden können und deren Beschichtungen extrem resistent gegen Chemikalien sind.

Überraschenderweise wurde gefunden, daß urethanisierte β-Hydroxyalkylamid-Verbindungen hervorragende Vernetzer darstellen und in Kombination mit sauren Polymeren in Pulverlacken eine drastische erhöhte Chemikalienfestigkeit bewirken, ohne daß Einbußen in der Flexibilität, der Härte, der Reaktivität und der Wetterbeständigkeit in Kauf genommen werden müssen.

Gegenstand der vorliegenden Erfindung sind daher urethanisierte β-Hydroxyalkylamid-Verbindungen, aufgebaut aus den Komponenten
A) 65 bis 96 Gew.-% β-Hydroxyalkylamid, und
B) 4 bis 35 Gew.-% eines nicht aromatischen Polyisocyanats mit einer NCO-Funktionalität ≥ 2,
wobei die urethanisierten β Hydroxyalkylamid-Verbindungen terminal Hydroxylgruppen tragen und eine Funktionalität ≥ 2 aufweisen.

Ein bevorzugter Gegenstand der Erfindung sind solche urethanisierte β-Hydroxyalkylamid-Verbindungen, wobei das β-Hydroxyalkylamid A) die Formel aufweist 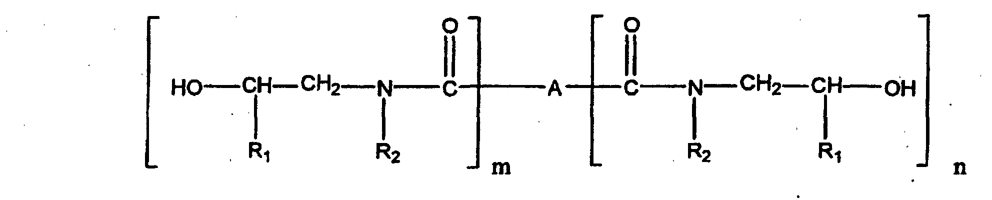 in der R₁ Wasserstoff oder eine C₁-C₅-Alkylgruppe, R₂ Wasserstoff, eine C₁-C₅-Alkylgruppe oder 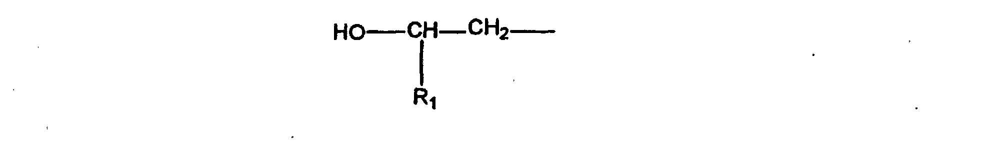 und A eine chemische Bindung oder eine einwertige oder mehrwertige organische Gruppe, ausgewählt aus gesättigten, ungesättigten oder aromatischen Kohlenwasserstoffgruppen, oder substituierten Kohlenwasserstoffgruppen, mit 2 bis 20 Kohlenstoffatomen, bedeuten, m ist 1 bis 2, n ist 0 bis 2 und m + n ist mindestens 2. Besonders bevorzugt weisen diese erfindungsgemäßen Verbindungen eine Funktionalität von vier oder mehr auf.

Weiterer Gegenstand der Erfindung ist auch die Verwendung der urethanisierten β-Hydroxyalkylamid-Verbindungen zur Herstellung transparenter oder pigmentierter außenbeständiger Pulverlacke hoher Reaktivität und Härte, ausgezeichnetem Glanz und sehr guter Chemikalienresistenz, hergestellt aus der urethanisierten β-Hydroxyalkylamid-Verbindung und carboxylgruppenhaltigen Polymeren sowie den in der Pulverlack-Chemie üblichen Zuschlagsstoffen wie z. B. Pigmente, Füllstoffe, Verlaufmittel, Entgasungsmittel, ggf. Katalysatoren und andere Additive.

Gegenstand der Erfindung sind auch transparente und pigmentierte Pulverlacke, welche die erfindungsgemäßen urethanisierten β-Hydroxyalkylamid-Verbindungen enthalten.

Die β-Hydroxyalkylamide A) sind prinzipiell bekannt und z. B. in US 4,076,917, US 4,101,606, EP 0 322 834 und EP 0 473 380 beschrieben. Die Struktur kann folgendermaßen beschrieben werden: 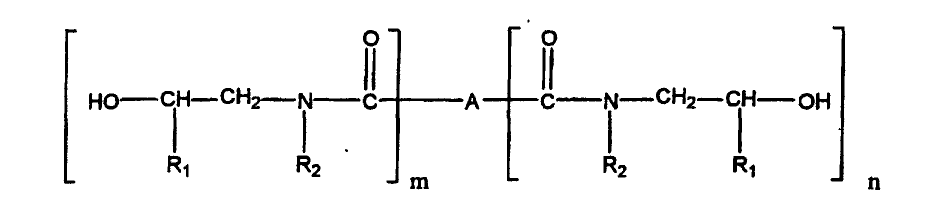 in der R₁ Wasserstoff oder C₁-C₅-Alkyl ist, R₂ ist Wasserstoff, C₁-C₅-Alkyl oder 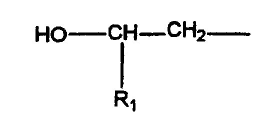 in der R₁ die zuvor angegebene Bedeutung hat und A ist eine chemische Bindung oder eine einwertige oder mehrwertige organische Gruppe, abgeleitet aus gesättigten, ungesättigten oder aromatischen Kohlenwasserstoffgruppen, einschließlich substituierter Kohlenwasserstoffgruppen mit 2 bis 20 Kohlenstoffatomen, m ist 1 bis 2, n ist 0 bis 2 und m + n ist mindestens 2.

Das nicht aromatische Polyisocyanat B) mit einer NCO-Funktionalität ≥ 2 kann jedes aliphatische, (cyclo)aliphatische, cycloaliphatische und heterocyclische Polyisocyanat mit mindestens zwei Isocyanatgruppen und Gemische hiervon sein. Derartige Polyisocyanate werden z. B. in Houben-Weyl, Methoden der Organischen Chemie, Band 14/2, Seite 61 ff. , und J. Liebigs Annalen der Chemie, Band 562, Seiten 75 bis 136, genannt. Repräsentative Beispiele der Polyisocyanate sind aliphatische Isocyanate wie Alkylenisocyanate, z. B. Ethylendiisocyanat, Propylendiisocyanat, Tetramethylendiisocyanat, Pentamethylendiisocyanat, 2-Methylpentamethylen-1,5-diisocyanat (MPDI), Hexamethylendiisocyanat (HDI), Trimethylhexamethylen-1,6-diisocyanat (TMDI), insbesondere das 2,2,4- und das 2,4,4-Isomere und technische Gemische beider Isomeren, Decamethylendiisocyanat und Dodecamethylendiisocyanat sowie Cycloalkylenisocyanate, z. B. 1,3-Cyclopentyldiisocyanat, 1,2-Cyclohexyldiisocyanat, 1,4-Cyclohexyldiisocyanat, ω,ω'-Diisocyanato- 1,4-methylcyclohexan, ω,ω'-Diisocyanato-1,3-dimethylcyclohexan, 1-Methyl-2,4-diisocyanatocyclohexan, 4,4'-Methylen-bis(cyclohexylisocyanat), Norbornandiisocyanat (NBDI) und 3,3,5-Trimethyl-1-isocyanato-3-isocyanatomethylcyclohexan (Isophorondiisocyanat, IPDI). Vorteilhaft sind Polyisocyanate, die durch Umsetzung von Polyisocyanaten mit sich selbst über Isocyanatgruppen erhältlich sind, insbesondere Isocyanurate, die durch Reaktion dreier Isocyanatgruppen entstehen. Gemische aus Diisocyanaten und Isocyanuraten, insbesondere aus 2-Methylpentamethylen-1,5-diisocyanat, 2,2,4-Trimethylhexamethylen-1,6-düsocyanat, 2,4,4-Trimethylhexamethylen-1,6-diisocyanat, Norbornandiisocyanat, Isophorondiisocyanat, Isocyanurat des 2-Methylpentamethylen- 1,5-diisocyanat, Isocyanurat des Hexamethylendiisocyanat oder Isocyanurat des Isophorondiisocyanat, sind besonders vorteilhaft. Die Polyisocyanate können ebenfalls Biuret- oder Allophanatgruppen enthalten.

Gegenstand der Erfindung ist auch ein Verfahren zur Herstellung von urethanisierten β-Hydroxyalkylamid-Verbindung, dadurch gekennzeichnet, daß 65 bis 96 Gew.-% mindestens eines β-Hydroxyalkylamid A) mit 4 bis 35 Gew.-% mindestens eines nicht aromatischen Polyisocyanats B) umgesetzt werden, die urethanisierten β-Hydroxyalkylamid-Verbindungen terminal Hydroxylgruppen tragen und eine Funktionalität ≥ 2 aufweisen.

Die Herstellung der erfindungsgemäßen urethanisierten β-Hydroxyalkylamid-Verbindungen kann in Lösemittel erfolgen Bevorzugt werden sie jedoch in Substanz, also lösemittelfrei, hergestellt. Dazu wird das β-Hydroxyalkylamid A) vorgelegt und das Polyisocyanat B) zugesetzt. Die Umsetzung kann über die Bestimmung der NCO-Zahl verfolgt werden und ist nach 30 Minuten bis 3 Stunden beendet. Zur Abkühlung, Zerkleinerung und Absackung werden bekannte Verfahren und Technologien verwendet.

Ein weiterer Gegenstand der vorliegenden Erfindung ist die Verwendung der erfindungsgemäßen urethanisierten β-Hydroxyalkylamid-Verbindungen zur Herstellung transparenter oder pigmentierter witterungsbeständiger Pulverlacke hoher Reaktivität und Härte und ausgezeichnetem Glanz.

Gegenstand der Erfindung sind auch transparente oder pigmentierte Pulverlacke, welche die erfindungsgemäßen urethanisierten β-Hydroxyalkylamid-Verbindungen und carboxylgruppenhaltigen Polymeren sowie den in der Pulverlack-Chemie üblichen Zuschlagstoffen wie z. B. Pigmente, Füllstoffe, Verlaufmittel, Entgasusngsmittel, ggf. Katalysatoren und andere Additive enthalten. Im Vergleich zu β-Hydroxyalkylamid-Vernetzern, die keine Urethangruppen aufweisen, zeichnen sich die aus den erfindungsgemäßen Pulverlacken hergestellten Beschichtungen durch eine stark verbesserte Chemikalienresistenz aus.

Als Reaktionspartner für die erfindungsgemäßen urethanisierten β-Hydroxyalkylamid-Verbindungen zur Herstellung von Pulverlacken kommen carboxylgruppenhaltige Polymere in Frage. Als Polymere können Polymerisate, Polykondensate und Polyadditionsverbindungen eingesetzt werden. Grundsätzlich kann jedes Polymer verwendet werden, das mindestens zwei Carboxylgruppen enthält und bei mindestens 60 °C schmilzt. Besonders bevorzugt sind im Rahmen der Erfindung Polycarboxylpolyester und Polycarboxylpolyacrylate.

Bei den carboxylgruppenhaltigen Polymeren handelt es sich bevorzugt um Polyesterpolycarbonsäuren, die aus Polyolen und Polycarbonsäuren bzw. deren Derivaten hergestellt werden. Der Schmelzbereich dieser sauren Polyester liegt in einem Bereich von 60 bis 160 °C, vorzugsweise 80 bis 120 °C; ihre Säurezahl variiert von 10 bis 150 mg KOH/g, vorzugsweise 30 bis 60 mg KOWg. Die OH-Zahlen sollen unter 10 mg KOH/g liegen.

Für die Herstellung der erfindungsgemäß zu verwendenden Polyesterpolycarbonsäuren werden Polycarbonsäure, wie z. B. Oxal-, Adipin-, 2,2,4(2,4,4)-Trimethyladipin-, Azelain-, Sebacin-, Decandicarbon-, Dodecandicarbon-, Fumar-, Phthal-, Isophthal-, Terephthal-, Trimellit-, Pyromellitsäure eingesetzt. Für die sauren Polyester werden als Polyole beispielhaft folgende verwendet: Ethylenglykol, 1,2-und 1,3-Propandiol, 1,2-, 1,3-, 1,4- und 2,3-Butandiol, 1,5-Pentandiol, 3-Methyl-1,5-pentandiol, Neopentylglykol, 1,12-Dodecandiol, 2,2,4(2,4,4)-Trimethyl-1,6-hexandiol, Trimethylolpropan, Glycerin, Pentaerythrit, 1,4-Bishydroxymethylcyclohexan, Cyclohexan-1,4-diol, Diethylenglykol, Triethylenglykol sowie Dipropylenglykol. Selbstverständlich können auch Hydroxylgruppen enthaltende Polyester, die nach bekannten Verfahren aus Polycarbonsäuren und Polyolen hergestellt werden, mit Polycarbonsäure und/oder Polycarbonsäureanhydriden zu den Polyesterpolycarbonsäuren umgesetzt werden.

Geeignete carboxylfunktionelle Acrylatpolymere besitzen eine Säurezahl von 10 bis 150 mg KOH/g und einen Schmelzpunkt von 60 bis 160 °C, hergestellt durch Copolymerisation eines Monomerengemisches, bestehend aus,
a) 0 bis 70 Gew.-Teilen Methyl(methacrylat),
b) 0 bis 60 Gew.-Teilen (Cyclo)Alkylestern der Acryl- und/oder Methacrylsäure mit 2 bis 18 Kohlenstoffatomen im Alkyl- bzw. im Cycloalkylrest,
c) 0 bis 90 Gew.-Teilen Vinylaromaten und
d) 0 bis 60 Gew.-Teilen olefinisch ungesättigten Carbonsäuren,
wobei die Summe der Gewichtsteile der Komponenten a) bis d) 100 ergibt.

Bei den Monomeren b) handelt es sich vorzugsweise um (Cyclo)Alkylester der Acryloder Methacrylsäure mit 2 bis 18 Kohlenstoffatomen im (Cyclo)Alkylrest. Beispiele geeigneter bzw. bevorzugt geeigneter Monomerer b) sind Ethyl(methyl)acrylat, n-Propyl(meth)acrylat, Isopropyl(meth)acrylat, n-Butyl(meth)acrylat, Isobutyl(meth)-acrylat, tert.-Butyl(meth)acrylat, 2-Ethylhexyl(meth)acrylat, Cyclohexylmethacrylat, Neopentylmethacrylat, Isobornylmethacrylat, 3,3,5-Trimethylcyclohexylmethacrylat und Stearylmethacrylat.

Als Monomere c) kommen z. B. Styrol, Vinyltoluol und Ethylstyrol in Betracht. Beispiele für d) sind Acryl- und Methacrylsäure, die auch bevorzugt eingesetzt werden, wie auch Crotonsäure, Itaconsäure, Fumarsäure, Maleinsäure und Citaconsäure.

Die Herstellung der Copolymerisate kann durch Copolymerisation der beispielhaft genannten Monomere a) bis d) nach üblichen radikalischen Polymerisationsverfahren erfolgen, wie beispielsweise Lösungs-, Emulsions-, Perl- oder Substanzpolymerisation. Dabei werden die Monomeren bei Temperaturen von 60 bis 160 °C, vorzugsweise 80 bis 150°C, in Gegenwart von Radikalbildnern und gegebenenfalls Molekulargewichtsreglern copolymerisiert.

Die Herstellung der carboxylfunktionellen Acrylat-Copolymerisate erfolgt in inerten Lösemitteln. Geeignete Lösemittel sind beispielsweise Aromaten, wie Benzol, Toluol, Xylol; Ester, wie Ethylacetat, Butylacetat, Hexylacetat, Heptylacetat, Methylglykolacetat, Ethylglykolacetat, Methoxypropylacetat; Ether, wie Tetrahydrofuran, Dioxan, Diethylenglykoldimethylether; Ketone, wie Aceton, Methylethylketon, Methylisobutylketon, Methyl-n-amylketon, Methylisoamylketon, oder beliebige Gemische derartiger Lösemittel.

Die Herstellung der Copolymerisate kann kontinuierlich oder diskontinuierlich erfolgen. Üblicherweise wird in einen Polymerisationsreaktor gleichmäßig und kontinuierlich die Monomermischung und der Initiator eindosiert und gleichzeitig die entsprechende Menge Polymerisat kontinuierlich abgeführt. Vorzugsweise können so chemisch nahezu einheitliche Copolymere hergestellt werden. Chemisch nahe einheitliche Copolymere können auch hergestellt werden, indem man die Reaktionsmischung mit konstanter Geschwindigkeit in einen Rührkessel einlaufen läßt, ohne das Polymerisat abzuführen.

Man kann auch einen Teil der Monomeren beispielhaft in Lösemitteln der genannten Art vorlegen und die restlichen Monomeren und Hilfsmittel getrennt oder gemeinsam in diese Vorlage bei der Reaktionstemperatur eintragen. Im Allgemeinen erfolgt die Polymerisation unter Atmosphärendruck, kann jedoch auch bei Drücken bis zu 25 bar durchgeführt werden. Die Initiatoren werden in Mengen von 0,05 bis 15 Gew.-%, bezogen auf die Gesamtmenge der Monomeren, eingesetzt.

Geeignete Initiatoren sind übliche Radikalstarter, wie z. B. aliphatische Azoverbindungen, wie Azodiisobuttersäurenitril, Azo-bis-2-methylvaleronitril, 1,1'-Azo-bis-1-cyclohexannitril und 2,2'-Azo-bis-isobuttersäurealkylester; symmetrische Diacylperoxide, wie z. B. Acetyl-, Propionyl- oder Butyrylperoxid, mit Brom-, Nitro-, Methyl- oder Methoxygruppen substituierte Benzoylperoxide, Laurylperoxide; symmetrische Peroxydicarbonate, z. B. tert.-Butylperbenzoat; Hydroperoxide, wie beispielsweise tert.-Butylhydroperoxid, Cumolhydroperoxid; Dialkylperoxide, wie Dicumylperoxid, tert.-Butylcumylperoxid oder Di-tert.-butylperoxid. Zur Regelung des Molekulargewichts der Copolymerisate können übliche Regler bei der Herstellung eingesetzt werden. Beispielhaft genannt seien Mercaptopropionsäure, tert.-Dodecylmercaptan, n-Dodecylmercaptan oder Diisopropylxanthogendisulfid. Die Regler können in Mengen von 0,1 bis 10 Gew.-%, bezogen auf die Gesamtmenge der Monomeren, zugegeben werden.

Die bei der Copolymerisation anfallenden Lösungen der Copolymerisate können dann ohne weitere Aufarbeitung dem Ausdampfungs- bzw. Entgasungsprozess zugeführt werden, bei dem das Lösemittel beispielsweise in einem Ausdampfextruder oder Sprühtrockner bei ca. 120 bis 160 °C und einem Vakuum von 100 bis 300 mbar entfernt und die erfindungsgemäß zu verwendenden Copolymerisate gewonnen werden.

Als Polycarboxylverbindungen können selbstverständlich auch Gemische mehrerer Stoffe eingesetzt werden.

Das Mischungsverhältnis der carboxylgruppenhaltigen Polymere und der erfindungsgemäßen urethanisierten β-Hydroxyalkylamid-Verbindung wird in der Regel so gewählt, daß das Verhältnis Carboxylgruppen zu Hydroxylgruppen 0,6 : 1 bis 1,6 : 1 beträgt.

In der Regel ist der Zusatz eines Katalysators zur Erhöhung der Geliergeschwindigkeit der hitzehärtbaren Pulverlacke, nicht erforderlich. Enthält das saure Polymer ein aliphatisches Harz, in dem Reste der 1,4-Cyclohexandicarbonsäure (CHDA) oder des 2,2,4,4-Tetramethyl-1,3-cyclobutanediols der 1,4-CHDA oder hydriertes Bisphenol A enthalten sind, können, wie in der WO 95/01406 beschrieben, Katalysatoren aus C₁-C₁₈ Zink-, Aluminium- oder Titan-Carboxylat-Salzen -Salzen oder Aluminium- oder Zinkoxiden beschleunigend wirken. Sie werden in Mengen von 0,03 bis 1,0 Gew.-%, bezogen auf die gesamte Pulvermenge, eingesetzt.

Die erfindungsgemäßen urethanisierten β-Hydroxyalkylamid-Verbindungen werden für die Herstellung von Pulverlacken mit dem geeigneten carboxylgruppenhaltigen Polymeren und gegebenenfalls Katalysatoren sowie Pigmenten und üblichen Hilfsmitteln wie Füllstoffen, Entgasungsmittteln und Verlaufmitteln gemischt. Alle Inhaltsstoffes des Pulverlackes werden in der Schmelze homogenisiert. Dies kann in geeigneten Aggregaten, wie z. B. beheizbaren Knetern, vorzugsweise jedoch durch Extrudieren, erfolgen, wobei Temperaturobergrenzen von 130 bis 140 °C nicht überschritten werden sollten. Die extrudierte Masse wird nach Abkühlen auf Raumtemperatur und nach geeigneter Zerkleinerung zum sprühfertigen Pulver vermahlen. Das Auftragen des sprühfertigen Pulvers auf geeignete Substrate kann nach den bekannten Verfahren, wie z. B. durch elektrostatisches oder tribostatisches Pulversprühen, Wirbelsintern, elektrostatisches Wirbelsintern, erfolgen. Nach dem Pulverauftrag werden die beschichteten Werkstücke zur Aushärtung 60 bis 5 Minuten auf eine Temperatur von 150 bis 220 °C erhitzt.

Die Erfindung wird nachfolgend anhand von Beispielen beschrieben.

### Beispiele

### A Herstellung der erfindungsgemäβen urethanisierten β-Hydroxyalkylamid-Verbindung

### A 1 β-Hydroxyalkylamid A)

Als β-Hydroxyalkylamid A) wurde das PRIMID XL-552 (OH-Zahl: 682 mg KOH/g, Schmelzpunkt: 125 °C, EMS-Chemie AG) eingesetzt.

### A 2 Polyisocyanat B)

Als Polyisocyanat B) wurden VESTANAT® T 1890 (Isocyanurat des IPDI, NCO-Zahl 17,1 %, CREANOVA Spezialchemie GmbH) und Isophorondiisocyanat (IPDI, NCO-Zahl 37,8 %, CREANOVA Spezialchemie GmbH) eingesetzt.

### A 3 Urethanisierte β-Hydroxyalkylamid-Verbindungen

### Allgemeine lösemittelfreie Herstellungsvorschrift

In einen Stahltopf wird das β-Hydroxyalkylamid A) vorgelegt und bei einer Temperatur von ca. 125 °C aufgeschmolzen. Das Polyisocyanat B) wird zudosiert. Nach einer Reaktionszeit von ca. 3 Stunden wird das Produkt abgekühlt und zerkleinert.

Die physikalischen und chemischen Kenndaten der erfinderischen Verfahrensprodukte sowie die molaren Zusammensetzungen sind in Tabelle 1 zusammengefaßt.

**Tabelle 1:**

| Urethanisierte β-Hydroxyalkylamide im Vergleich zum β-Hydroxyalkylamid | | | | | |
|---|---|---|---|---|---|
| Beispiele Nr. | Zusamensetzung | | | Kenndaten | |
| | PRIMID XL-552 [Gew.-%] | T 1890 [Gew.-%] | IPDI (Gew.-%] | OH-Zahl [mgKOH/g] | Smp. [°C} |
| A 3.1 | 72,9 | 27,1 | - | 435 | 93-98 |
| A3.2 | 80,2 | 19,8 | - | 501 | 104-110 |
| A 3.3 | 84,4 | 15,6 | - | 539 | 109-113 |
| A 3.4 | 73,9 | 24,8 | 1,3 | 441 | 90-95 |
| A 3.5 Vergleich | 100 | - | - | 682 | 125 |

### B Polymer

Als Polymer wurde der saure Polyester Grilesta P 7617 der Fa. EMS-Chemie AG (Säurezahl 35 mg KOH/g, Tg 61 °C, Viskosität bei 200 °C 3 500 mPa·s) eingesetzt.

### C Pulverlacke

### Allgemeine Herstellungsvorschrift

Die zerkleinerten Produkte, d. h. urethanisierte β-Hydroxyalkylamid-Verbindung, saurer Polyester, Verlaufmittel-Masterbatch, ggf. Katalysatoren, werden ggf. mit dem Weißpigment in einem Kollergang innig vermischt und anschließend in einem Zweischneckenextruder der Fa. Berstorff bis maximal 130 °C homogenisiert. Nach dem Erkalten wird das Extrudat gebrochen und mit einer Stiftmühle auf eine Korngröße < 100 µm gemahlen. Das so hergestellte Pulver wird mit einer elektrostatischen Pulverspritzanlage bei 60 KV auf entfettete, ggf. vorbehandelte Eisenbleche appliziert und in einem Umlufttrockenschrank bei Temperaturen zwischen 150 und 220 °C eingebrannt.

### Verlaufmittel-Masterbatch

Es werden 10 Gewichtsprozent des Verlaufimittels - ein handelsübliches Copolymer von Butylacrylat und 2-Ethylhexylacrylat - in dem entsprechenden Polyester in der Schmelze homogenisiert und nach dem Erstarren zerkleinert.

Die Abkürzungen in den folgenden Tabellen bedeuten:

| | |
|---|---|
| SD = | Schichtdicke in µm |
| HK = | Härte nach König (sec) (DIN 53 157) |
| ET = | Tiefung nach Erichsen (DIN 53 156) |
| GS = | Gitterschnittprüfung (DIN 53 151) |
| GG 60 °-Winkel = | Messung des Glanzes n. Gardner (ASTM-D 5233) |
| Imp rev. = | Impact reverse in inch·lb |
| MEK-Test = | Methylethylketon-Test in Hüben |
| (ein mit MEK getränkter gepreßter Wattebausch wird mit einem 1 kg schweren Hammer über die Beschichtung geführt bis sie matt wird.) | |

**Tabelle 2:**

| **Pigmentierte Pulverlacke** | | | | | |
|---|---|---|---|---|---|
| Beispiel C Rezeptur | 1 | 2 | 3 | 4 | 5 Vergleich |
| Vernetzer gem. A 3 | 6,86 | 6,53 | 6,09 | 7,35 | 4,84 |
| Tabelle 1 | (1) | (2) | (3) | (4) | (5) |
| Polyester gem. B | 93,14 | 93,47 | 93,91 | 92,65 | 95,16 |
| Bemerkungen: | 35 Gew.-% TiO₂ (Weißpigment), 1,0 Gew.-% Resiflow PV 88, 0,3 Gew.-% Benzoin, COOH/OH-Verhältnis =1:1 | | | | |
| Lack-Daten | | | | | |
| SD | 50-64 | 70-94 | 62-96 | 55-70 | 81-101 |
| HK | 174 | 192 | 195 | 192 | 193 |
| GS | 0 | 0 | 0 | 0 | 0 |
| GG 60 °-Winkel | 92 | 92 | 93 | 91 | 92 |
| ET | > 10 | > 10 | > 10 | > 10 | > 10 |
| Imp.Rev. | >80 | >80 | > 80 | >80 | >80 |
| MEK-Test | 32 | 50 | 50 | 46 | 16 |
| Härtung: | 200 °C/10 Minuten | | | | |

## Patentansprüche

1. Urethanisierte β-Hydroxyalkylamid-Verbindungen, aufgebaut aus den Komponenten
A) 65 bis 96 Gew.-% β-Hydroxyalkylamid, und
B) 4 bis 35 Gew.-% eines nicht aromatischen Polyisocyanats mit einer NCO-Funktionalität ≥ 2,
wobei die urethanisierten β-Hydroxyalkylamid-Verbindungen terminal Hydroxylgruppen tragen und eine Funktionalität ≥ 2 aufweisen.

2. Urethanisierte β-Hydroxyalkylamid-Verbindungen nach Anspruch 1,
**dadurch gekennzeichnet,**
**daß** das β-Hydroxyalkylamid A) die Formel aufweist 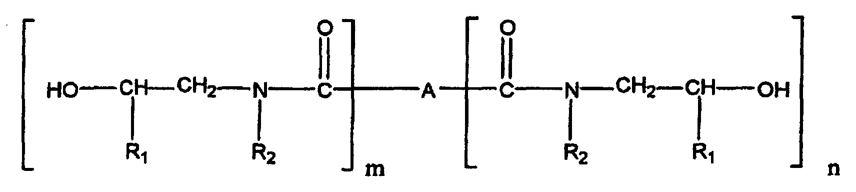 in der R₁ Wasserstoff oder eine C₁-C₅-Alkylgruppe, R₂ Wasserstoff, eine C₁-C₅-Alkylgruppe oder 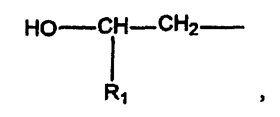 und A eine chemische Bindung oder eine einwertige oder mehrwertige organische Gruppe, ausgewählt aus gesättigten, ungesättigten oder aromatischen Kohlenwasserstoffgruppen, oder substituierten Kohlenwasserstoffgruppen mit 2 bis 20 Kohlenstoffatomen, bedeuten, m ist 1 bis 2, n ist 0 bis 2 und m + n ist mindestens 2.

3. Urethanisierte β-Hydroxyalkyiamid-Verbindungen nach Anspruch 1 oder 2, wobei diese eine Funktionalität ≥ 4 aufweisen.

4. Urethanisierte β-Hydroxyalkylamid-Verbindung nach mindestens einem der Ansprüche 1 bis 3,
**dadurch gekennzeichnet,**
**daß** als Polyisocyanat B) mindestens eine Verbindung aus der Gruppe der aliphatischen, (cyclo)aliphatischen, cycloaliphatischen oder heterocyclischen Polyisocyanate mit mindestens zwei Isocyanatgruppen ausgewählt wird.

5. Urethanisierte β-Hydroxyalkylamid-Verbindung nach mindestens einem der Ansprüche 1 bis 4,
**dadurch gekennzeichnet,**
**daß** als Polyisocyanat B) mindestens eine Verbindung ausgewählt aus Ethylendiisocyanat, Propylendiisocyanat, Tetramethylendiisocyanat, Pentamethylendiisocyanat, 2-Methylpentamethylen-1,5-diisocyanat (MPDI), Hexamethylendiisocyanat(HDI), Trimethylhexamethylen-1,6-diisoyanat (TMDI), insbesondere das 2,2,4- und das 2,4,4-Isomere und technische Gemische beider Isomeren, Decamethylendiisocyanat und Dodecamethylendiisocyanat, 1,3-Cyclopentyldiisocyanat, 1,2-Cyclohexyldiisocyanat, 1,4-Cyclohexyldiisocyanat, ω,ω' -Diisocyanato-1,4-methylcyclohexan, ω,ω' -Diisocyanato-1,3-dimethylcyclohexan, 1-Methyl-2,4-diisocyanatocyclohexan, 4,4'-Methylenbis(cyclohexylisocyanat), Norbornandiisocyanat (NBDI), 3,3,5-Trimethyl-1-isocyanato-3-isocyanatomethylcclohexan (Isophorondiisocyanat IPDI) eingesetzt wird.

6. Urethanisierte β-Hydroxyalkylamid-Verbindung nach mindestens einem der Ansprüche 1 bis 5,
**dadurch gekennzeichnet,**
**daß** als Polyisocyanat B) beliebige Gemische aus 2-Methylpentamethylen-1,5-diisocyanat, 2,2,4-Trimethylhexamethylen-1,6-diisocyanat, 2,4,4-Trimethyl-hexamethylen-1,6-diisocyanat, Norbornandiisocyanat, Isophorondiisocyanat, Isocyanurat des 2-Methylpentamethylen-1,5-diisocyanat, Isocyanurat des Hexamethylendiisocyanat oder Isocyanurat des Isophorondiisocyanat eingesetzt wird.

7. Verfahren zur Herstellung von urethanisierten β-Hydroxyalkylamid-Verbindungen nach einem der Ansprüche 1bis 6,
**dadurch gekennzeichnet,**
**daß** 65 bis 96 Gew.-% mindestens eines β-Hydroxyalkylamid A) mit 4 bis 35 Gew.-% mindestens eines nicht aromatischen Polyisocyanats B) umgesetzt werden, die urethanisierten β-Hydroxyalkylamid-Verbindungen terminal Hydroxylgruppen tragen und eine Funktionalität ≥ 2 aufweisen.

8. Verfahren zur Herstellung der urethanisierten β-Hydroxyalkylamid-Verbindung nach Anspruch 7,
**dadurch gekennzeichnet,**
**daß** die Umsetzung der Verbindungen A) und B) lösemittelfrei in einem Batchprozeß erfolgt.

9. Verwendung der urethanisierten β-Hydroxyalkylamid-Verbindung nach den Ansprüchen 1 bis 8,
in Kombination mit carboxylgruppenhaltigen Polymeren zur Herstellung transparenter oder pigmentierter witterungsbeständiger Pulverlacke mit sehr guter Chemikalienresistenz.

10. Transparente oder pigmentierte Pulverlacke,
**dadurch gekennzeichnet,**
**daß** diese mindestens eine urethanisierte β-Hydroxyalkylamid-Verbindung gemäß den Ansprüchen 1 bis 6, in Kombination mit carboxylgruppenhaltigen Polymeren sowie weiteren Hilfs-und Zuschlagstoffe, enthalten.

11. Transparente oder pigmentierte Pulverlacke nach Anspruch 10,
**dadurch gekennzeichnet,**
**daß** als Hilfs- und Zuschlagstoffe Füllstoffe, Verlaufmittel, Entgasungsmittel oder Katalysatoren enthalten sind.

12. Transparente oder pigmentierte Pulverlacke nach den Ansprüchen 10 bis 11,
**dadurch gekennzeichnet,**
**daß** ein COOH/OH-Verhältnis von 0,6 : 1,0 bis 1,6 : 1,0 zugrunde liegt.

13. Transparente oder pigmentierte Pulverlacke nach Anspruch 12,
**dadurch gekennzeichnet,**
**daß** ein COOH/OH-Verhältnis von 0,8:1,0 bis 1,2:1,0 zugrunde liegt.

14. Transparente oder pigmentierte Pulverlacke nach Anspruch 12,
**dadurch gekennzeichnet,**
**daß** ein COOH/OH-Verhältnis von 1 : 1 bis 1:1 zugrunde liegt.

15. Transparente oder pigmentierte Pulverlacke nach den Ansprüchen 10 bis 14,
**dadurch gekennzeichnet,**
**daß** die Pulverlacke Katalysatoren in einer Konzentration von 0,03 bis 1,0 Gew.-%, bezogen auf die gesamte Pulverlackmenge, enthalten.

16. Transparente oder pigmentierte Pulverlacke nach Anspruch 15,
**dadurch gekennzeichnet,**
**daß** die Pulverlacke als Katalysatoren Aluminium-Carboxylat-Salze, Titan-Carboxylat-Salze, Aluminiumoxide oder Zinkoxide enthalten.

17. Transparente oder pigmentierte Pulverlacke nach den Ansprüchen 10 bis 16,
**dadurch gekennzeichnet,**
**daß** als carboxylgruppenhaltige Polymere Polycarboxylpolyester oder Polycarboxylpolyacrylate eingesetzt werden.

18. Transparente oder pigmentierte Pulverlacke nach Anspruch 17,
**dadurch gekennzeichnet,**
**daß** die Polycarboxylpolyester eine Säurezahl von 10 bis 150 mg KOH/g, und einen Schmelzpunkt von 60 bis 160 °C aufiveisen.

19. Transparente oder pigmentierte Pulverlacke nach Anspruch 17,
**dadurch gekennzeichnet,**
**daß** die Polycarboxylpolyacrylate eine Säurezahl von 10 bis 150 mg KOH/g und einen Schmelzpunkt von 60 bis 160 °C aufweisen.

## Claims

1. A urethanized β-hydroxyalkylamide compound synthesized from the components
A) from 65 to 96% by weight of β-hydroxyalkylamide and
B) from 4 to 35% by weight of a nonaromatic
polyisocyanate having an NCO functionality ≥ 2, the urethanized β-hydroxyalkylamide compound carrying hydroxyl groups terminally and having a functionality ≥ 2.

2. A urethanized β-hydroxyalkylamide compound according to claim 1, **characterized in that** the β-hydroxyalkylamide A) has the formula 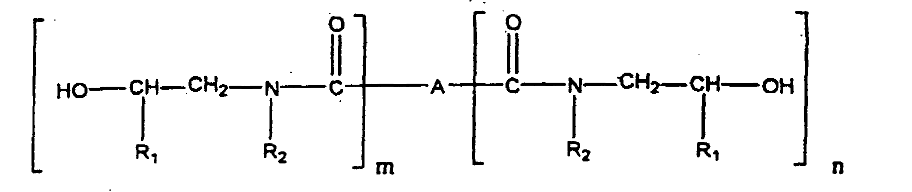 in which R₁ is hydrogen or a C₁-C₅ alkyl group, R₂ is hydrogen, a C₁-C₅ alkyl group or 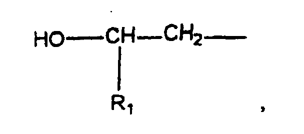 and A is a chemical bond or a monovalent or polyvalent organic group selected from saturated, unsaturated and aromatic hydrocarbon groups, and substituted hydrocarbon groups, having 2 to 20 carbon atoms, m is 1 or 2, n is from 0 to 2 and m + n is at least 2.

3. A urethanized β-hydroxyalkylamide compound according to either of claims 1 and 2, which has a functionality ≥ 4.

4. A urethanized β-hydroxyalkylamide compound according to at least one of claims 1 to 3, **characterized in that** the polyisocyanate B) comprises at least one compound from the group consisting of aliphatic, (cyclo)aliphatic, cycloaliphatic and heterocyclic polyisocyanates having at least two isocyanate groups.

5. A urethanized β-hydroxyalkylamide compound according to at least one of claims 1 to 4, **characterized in that** the polyisocyanate B) comprises at least one compound selected from ethylene diisocyanate, propylene diisocyanate, tetramethylene diisocyanate, pentamethylene diisocyanate, 2-methylpentamethylene 1,5-diisocyanate (MPDI), hexamethylene diisocyanate (HDI), trimethylhexamethylene 1,6-diisocyanate (TMDI), especially the 2,2,4 and the 2,4,4 isomers and technical-grade mixtures of both isomers, decamethylene diisocyanate and dodecamethylene diisocyanate, 1,3-cyclopentyl diisocyanate, 1,2-cyclohexyl diisocyanate, 1,4-cyclohexyl diisocyanate, ω,ω' -diisocyanato-l, 4-methylcyclohexane, ω,ω' -diisocyanato1, 3.-dimethylcyclohexane, 1-methyl-2,4-diisocyanatocyclohexane, 4,4'-methylenebis(cyclohexyl isocyanate), norbornane diisocyanate (NBDI) and 3, 3, 5-trimethyl-1-isocyanato-3-isocyanatomethylcyclohexane (isophorone diisocyanate, IPDI).

6. A urethanized β-hydroxyalkylamide compound according to at least one of claims 1 to 5, **characterized in that** said polyisocyanate B) comprises any desired mixtures of 2-methylpentamethylene 1,5-diisocyanate, 2,2,4-trimethylhexamethylene 1,6-diisocyanate, 2,4,4-trimethylhexamethylene 1,6-diisocyanate, norbornane diisocyanate, isophorone diisocyanate, the isocyanurate of 2-methylpentamethylene 1,5-diisocyanate, the isocyanurate of hexamethylene diisocyanate or the isocyanurate of isophorone diisocyanate.

7. A process for preparing a urethanized β-hydroxyalkylamide compound according to any one of claims 1 to 6, **characterized in that** it comprises reacting from 65 to 96% by weight of at least one β-hydroxyalkylamide A) with from 4 to 35% by weight of at least one nonaromatic polyisocyanate B), the urethanized β-hydroxyalkylamide compound carrying hydroxyl groups terminally and having a functionality ≥ 2.

8. A process for preparing a urethanized β hydroxyalkylamide compound according to claim 7, **characterized in that** the reaction of the compounds A) and B) takes place without solvent in a batch process.

9. The use of a urethanized β-hydroxyalkylamide compound according to any of claims 1 to 8 in combination with carboxyl-containing polymers to prepare transparent or pigmented weathering-resistant powder coating materials having very good chemical resistance.

10. A transparent or pigmented powder coating material **characterized in that** it comprises at least one urethanized β-hydroxyalkylamide compound according to any of claims 1 to 6 in combination with carboxyl-containing polymers and also further auxiliaries and adjuvants.

11. A transparent or pigmented powder coating material according to claim 10, **characterized in that** said auxiliaries and adjuvants comprise fillers, levelling agents, devolatilizers or catalysts.

12. A transparent or pigmented powder coating material according to either of claims 10 and 11, **characterized in that** it is based on a COOH/OH ratio of from 0.6:1.0 to 1.6:1.0.

13. A transparent or pigmented powder coating material according to claim 12, **characterized in that** it is based on a COOH/OH ratio of from 0.8:1.0 to 1.2:1.0.

14. A transparent or pigmented powder coating material according to claim 12, **characterized in that** it is based on a COOH/OH ratio of 1:1.

15. A transparent or pigmented powder coating material according to any of claims 10 to 14, **characterized in that** it comprises catalyst(s) in a concentration of from 0.03 to 1.0% by weight, based on the total amount of powder coating material.

16. A transparent or pigmented powder coating material according to claim 15, **characterized in that** it comprises as catalyst(s) aluminium carboxylate salts, titanium carboxylate salts, aluminium oxides or zinc oxides.

17. A transparent or pigmented powder coating material according to any of claims 10 to 16, **characterized in that** polycarboxyl polyesters or polycarboxyl polyacrylates are used as carboxyl-containing polymers.

18. A transparent or pigmented powder coating material according to claim 17, **characterized in that** the polycarboxyl polyesters have an acid number of from 10 to 150 mg KOH/g and a melting point of from 60 to 160°C.

19. A transparent or pigmented powder coating material according to claim 17, **characterized in that** the polycarboxyl polyacrylates have an acid number of from 10 to 150 mg KOH/g and a melting point of from 60 to 160°C.

## Revendications

1. Composés de β-hydroxyalkylamide uréthanisés constitués des composants
A) de 65 à 96 % en poids de β-hydroxyalkylamide,
et
B) de 4 à 35 % en poids d'un polyisocyanate non aromatique ayant une fonctionnalité en NCO ≥ 2
dans lesquels les composés de β-hydroxyallylamide uréthanisés portent des groupes hydroxyle terminaux et possèdent une fonctionnalité ≥ 2.

2. Composés de β-hydroxyalhylamide uréthanisés selon la revendication 1,
**caractérisés en ce que**
le β-hydroxyalkylamide A possède la formule 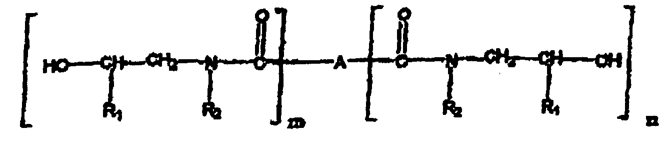 dans laquelle
R₁ signifie de l'hydrogène ou un groupe C₁-C₅-alkyle, R₂ de l'hydrogène, un groupe allyle en C₁-C₅ ou 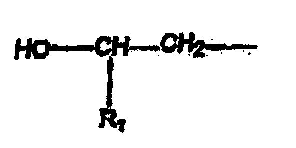 et A une liaison chimique ou un groupe organique monovalent ou plurivalent choisi parmi les groupes hydrocarbonés saturés, non saturés ou aromatiques, ou des groupes hydrocarbonés substitués ayant de 2 à 20 atomes de carbone, m est 1 à 2, n est 0 à 2 et m + n est au moins 2.

3. Composés de β-hydroxyalkylamide uréthanisés selon la revendication 1 ou 2,
dans lesquels
ceux-ci possèdent une fonctionnalité ≥ 4.

4. Composés de β-hydroxyalkylamide uréthanisés selon l'une des revendications 1 à 3,
**caractérisés en ce que**
comme polyisocyanate B) on sélectionne au moins un composé choisi dans le groupe des polyisocyanates aliphatiques, (cyclo)aliphatiques, Cycloaliphatiques ou hétérocycliques ayant au moins deux groupes isocyanate.

5. Composés de β-hydroxyalkylamide uréthanisés selon au moins une des revendications 1 à 4,
**caractérisés en ce que**
comme polyisocyanate B) on met en oeuvre au moins un composé choisi parmi l'éthylènediisocyanate, le propylènediisocyanate, le tétraméthylènediisocyanate, le pentaméthylènediisocyanate, le 2-méthylpentaméthylène-1,5-diisocyanate (MPDI), l'hexaméthylènediisocyanate (HDI), le triméthylhexaméthylène-1,6-diisocyanate (TMDI), en particulier l'isomère-2,2,4- et l'isomère-2,4,4 et des mélanges techniques des deux isomères, le décamèthylènediisocyanate, le dodécaméthylènediisocyanate, le 1,3-cyclopentyldiisocyanate, le 1,2-cyclohexyldiisocyanate, le 1,4-cyclohexyldiisocyanate, le ω , ω'-diisocyanato-1,4-méthylcyclohexane, le ω, ω'-diisocyanato- 1 ,3-diméthylcyclohexane, le 1-méthyl-2,4-diisocyanatocyclohexane, le 4,4'-méthylène-bis(cyclohexylisocyanate, le norbornanediisocyanate (NBDI), le 3,3,5-triméthyl-1-isocyanato-3-isocyanatométhylcyclohexane (isophoronediisocyanate IPDI).

6. Composés de β-hydroxyalkylamide uréthanisés selon au moins l'une des revendications 1 à 5,
**caractérisés en ce que**
comme polyisocyanate B) on met en oeuvre des mélanges quelconques à base de 2-méthylpentaméthylène-1,5-diisocyanate, 2,2,4-triméthylhexaméthylène-1,6-diisocyanate, 2,4,4-trimethylhexaméthylene- 1,6-diisocyanate, norbornanediisocyanate, isophoronediisocyanate, isocyanurate du 2-méthylpentaméthylène-1,5-disocyanate, isocyanurate du hexaméthylènediisocyanate ou l'isocyanurate de l'isophoronediisocyanate.

7. Procédé d'obtention de composés de β-hydroxyalkylamide uréthanisés selon l'une des revendications 1 à 6,
**caractérisé en ce qu'**
on met à réagir de 65 à 96 % en poids d'au moins un β-hydroxyalkylamide A) avec de 4 à 35 % en poids d'au moins un polyisocyanate B) non aromatique, qui portent des composés de β-hydroxyalkylamide uréthanisés d'une manière terminale et qui possèdent une fonctionnalité ≥ 2.

8. Procédé de préparation de composés de β-hydroxylalkylamide uréthanisé selon la revendication 7,
**caractérisé en ce que**
la réaction des composés A) et B) s'effectue sans solvant dans un processus par lots.

9. Utilisation du composé de β-hydroxyalkylamide uréthanisé selon les revendications 1 à 8,
en combinaison avec des polymères contenant des groupes carboxyle en vue de la production de laques en poudre résistantes aux intempéries, transparentes ou pigmentées, ayant une très bonne résistance aux produits chimiques.

10. Laques en poudre transparentes ou pigmentées,
**caractérisées en ce que**
celles-ci renferment au moins un composé de β-hydroxyalkylamide uréthanisé conformément aux revendications 1 à 6, en combinaison avec des polymères contenant des groupes carboxyle ainsi que d'autres adjuvants et additifs.

11. Laques en poudre transparentes ou pigmentées selon la revendication 10,
**caractérisées en ce que**
comme adjuvants et additifs, des substances de charge, des agents d'écoulement, des agents de dégazage ou des catalyseurs sont présents.

12. Laques en poudre transparentes ou pigmentées selon les revendications 10 à 11,
**caractérisées en ce qu'**
elles prennent pour base un rapport COOH/OH de 0,6 : 1,0 à 1,6 : 1,0.

13. Laques en poudre transparentes ou pigmentées selon la revendication 12,
**caractérisées en ce qu'**
elles prennent pour base un rapport COOH/OH de 0,8: 1,0 à 1,2 : 1,0.

14. Laques en poudre transparentes ou pigmentées selon la revendication 12,
**caractérisées en ce qu'**
elles reposent sur un rapport COOH/OH de 1 : 1.

15. Laques en poudre transparentes ou pigmentées selon les revendications 10 à 14,
**caractérisées en ce que**
les laques en poudre renferment des catalyseurs à une concentration allant de 0,03 à 1,0 % en poids, rapporté à la quantité totale de laque en poudre.

16. Laques en poudre transparentes ou pigmentées selon la revendication 15,
**caractérisées en ce que**
les laques en poudre renferment comme catalyseurs des sels de carboxylate d'aluminium, des sels de carboxylate de titane, des oxydes d'aluminium ou des oxydes de zinc.

17. Laques en poudre transparentes ou pigmentées selon les revendications 10 à 16,
**caractérisées en ce qu'**
on met en oeuvre comme polymères contenant des groupes carboxyle, des polyesters de polycarboxyle ou des polyacrylates de polycarboxyle.

18. Laques en poudre transparentes ou pigmentées selon la revendication 17,
**caractérisées en ce que**
les polyesters de polycarboxyle possèdent un indice d'acidité de 10 à 150 mg de KOH/g et un point de fusion de 60 à 160°C.

19. Laques en poudre transparentes ou pigmentées selon la revendication 17,
**caractérisées en ce que**
les polyacrylates de polycarboxyle possèdent un indice d'acidité de 10 à 150 mg KOH/g et un point de fusion de 60 à 160°C.
